# EUROPÄISCHE PATENTSCHRIFT

(11) **EP 2 347 302 B1**
(45) Veröffentlichungstag und Bekanntmachung des Hinweises auf die Patenterteilung: **09.08.2017**
(21) Anmeldenummer: 09744064.8
(22) Anmeldetag: 16.10.2009
(51) Int. Cl.: G03F 7/00, G03F 7/004, G02B 5/00, G02B 5/32, C07F 7/00, C07C 31/28

(54) **ZUSAMMENSETZUNG ZUR HERSTELLUNG OPTISCHER ELEMENTE MIT GRADIENTENSTRUKTUR**
COMPOSITION FOR PRODUCING OPTICAL ELEMENTS HAVING GRADIENT STRUCTURE
COMPOSITION POUR LA FABRICATION D'ÉLÉMENTS OPTIQUES AYANT UNE STRUCTURE EN GRADIENT

(30) Priorität: 21.10.2008 DE 102008052586
(43) Veröffentlichungstag der Anmeldung: 27.07.2011
(73) Patentinhaber: Leibniz-Institut für Neue Materialien gemeinnützige GmbH, 66123 Saarbrücken (DE)
(72) Erfinder: DE OLIVEIRA, Peter, William, 66123 Saarbrücken (DE); KÖNIG, Peter, 66822 Lebach (DE); VEITH, Michael, 66386 St.-Ingbert (DE); YAZDANI-ASSL, Omid, 66111 Saarbrücken (DE)
(74) Vertreter: Patentanwälte Gierlich & Pischitzis Partnerschaft mbB
(86) Internationale Anmeldenummer: PCT/EP2009/007458
(87) Internationale Veröffentlichungsnummer: WO 2010/046066

(56) Entgegenhaltungen:
- WO-A-2004/077530
- DE-A1-102004 061 323
- DE-A1-102006 011 949
- GB-A- 734 113
- JP-A- 2007 298 841
- US-A- 3 098 863
- US-A- 5 942 376
- US-A1- 2003 190 820

## Beschreibung

### Gebiet der Erfindung

Die Erfindung betrifft eine Zusammensetzung zur Herstellung optischer Elemente mit Gradientenstruktur, insbesondere für holographische Anwendungen, wobei die Gradientenstruktur durch einen Brechzahlgradienten gebildet wird, sowie ein Verfahren zur Herstellung dieser optischen Elemente mit Gradientenstruktur.

### Stand der Technik

Aus den US-Patenten 5,552,261 und 5,529,473 ist bekannt, die Diffusion von Monomeren, die gegenüber einer umgebenden flüssigen Matrix einen erhöhten oder verringerten Brechungsindex aufweisen, zur Erzeugung eines Brechzahlgradienten zu nutzen. Dieser bei Polymeren als Colburn-Haines-Effekt bekannte Effekt kann nach anschließender Polymerisation zu einem Produkt mit Brechzahlgradienten führen. Solche Polymere werden auch als Photopolymere bezeichnet. Zur Herstellung des Brechzahlengradienten wird eine lokale Polymerisation induziert, welche zu einer Potentialdifferenz führt. Diese begünstigt die Diffusion von weiteren Monomeren zum Ort der lokalen Polymerisation. Dies verändert den Brechungsindex an dieser Stelle im Vergleich zur direkten Umgebung.

Die Erzeugung von scharfen Brechzahlgradienten durch den Colburn-Haines-Effekts erfordert daher nicht nur die effiziente Diffusion der Monomere, sondern auch eine schnelle und effiziente Abreaktion der Monomere. Die Matrix ist dabei meistens ein Polymer, welches beispielsweise in einem Lösungsmittel gelöst ist. Wichtig ist, dass die Reaktion der Monomere die Matrix nicht beeinflussen darf. Außerdem sollte die Ausbildung des Brechzahlengradienten nicht zur Veränderung des gesamten Materials führen, beispielsweise durch Schrumpfung. Dies ist insbesondere bei der Erzeugung von Brechzahlgradienten in sehr dicken Schichten wichtig.

Ein Problem bei der Erzeugung von Brechzahlgradienten auf der Basis von rein organischen Systemen ist die begrenzte Bandbreite der realisierbaren Brechzahlen. Eine Möglichkeit die Bandbreite zu erhöhen ist die Verwendung von anorganischen Komponenten.

So wird beispielsweise in der Anmeldung US 2005/0101698 in einem Kompositmaterial ein Konzentrationsgradient von Nanopartikeln erzeugt. Dieses Verfahren erlaubt die Herstellung von Volumenhologrammen mit einer Brechungseffizienz von 90 %. Allerdings ist die Dicke der Schichten begrenzt und das Material zeigt eine hohe Schrumpfung wegen der radikalischen Polymerisation. Außerdem begrenzt die langsame Diffusion der Nanopartikel die möglichen Brechzahlen und einsetzbaren Matrizes.

Zusammenfassend ist festzustellen, dass die Entwicklung von Photopolymeren in den letzten Jahren große Fortschritte zu verzeichnen hatte. Dennoch besitzen die bekannten Systeme noch einige Nachteile. So sind die verwendeten Systeme nicht empfindlich genug, um eine sehr scharfe Modulation der Brechzahl zu ermöglichen. Dabei beinhaltet die Empfindlichkeit die zur Polymerisation benötigte Lichtstärke, als auch die zur Herstellung der Gradientenstruktur benötigte Belichtungsdauer. Beide begrenzen die Auflösung und Brechungseffizienz der hergestellten Gradientenstrukturen. Gleichzeitig spielt auch die im Material erzielte Bandbreite der Brechzahlmodulation eine wichtige Rolle. Beide genannten Parameter beispielsweise begrenzen die minimale Schichtdicke des Materials, mit dem die Herstellung von optischen Gradientenstrukturen noch möglich ist.

Da optische Gradientenstrukturen heutzutage in vielen Bereichen mit höchst unterschiedlichen Anforderungen, beispielsweise bezüglich mechanischer Flexibilität, Dicke und Stabilität, eingesetzt werden, ist eine hohe Variabilität der eingesetzten Komponenten von großer Bedeutung.

DE 10 2004 061 323 A1 beschreibt ein anorganisch-organisches Hybridmaterial zur Herstellung von Brechzahlgradientenschichten, bei dem Metallkomplexe mit polymerisierbaren Liganden lokal polymerisiert werden, um einen Brechzahlgradienten zu erzeugen.

DE 10 2006 011 949 A1 beschreibt die Verwendung von Metallkomplexen mit polymerisierbaren Liganden zur Herstellung von diffraktiven Lichtlenkelementen durch ein Rolle-zu-Rolle-Verfahren.

JP 2007-298841 A beschreibt eine lichtempfindliche Polymerzusammensetzung erhalten aus käfigförmigen Silikonen, einem Metallkomplex und einem organischen Lösungsmittel.

US 5,942,376 beschreibt einen lichtempfindlichen Metallkomplex zur Herstellung von dünnen Oxidschichten.

WO 2004/077530 A2 beschreibt lichtempfindliche Titaniumcarboxylate zur Verwendung in der Halbleiterherstellung.

GB 734113 beschreibt Komplexe von Titan mit α-Hydroxyketonen.

US 2004/0034245 A1 beschreibt Chelatkomplexe von Zirkonium mit β-Diketonen.

GB 1169011 beschreibt Komplexe von Titan mit Titan mit Hydroxybenzophenonen.

US 2003/0190820 A1 beschreibt lichtempfindliche Titancarboxylate.

US 3,098,863 beschreibt Metallkomplexe mit Hydroxybenzophenonen.

### Aufgabe

Aufgabe der Erfindung ist es, ein einfaches, universell anwendbares und kostengünstiges Verfahren zur Herstellung von optischen Elementen mit Gradientenstruktur bereitzustellen, welches die angegebenen Nachteile des Stands der Technik überwindet.

### Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht. Die Erfindung umfasst auch alle erwähnten Kombinationen von unabhängigen und/oder abhängigen Ansprüchen.

Die Aufgabe wird überraschenderweise von einer Zusammensetzung gelöst, welche mindestens ein organisches Polymer und mindestens einen Metallkomplex enthält, welcher mindestens eine lichtempfindliche Gruppe enthält, wobei der mindestens eine Metallkomplex der Formel

X₍ₘ₋ₙ₎MR¹ₙ (I)

ist,
wobei M für Ti, Zr, Ta, V, Nb, Cr, Mo, W, Mn oder Re steht; R¹ von einem α-Hydroxyketon, Glyoxylat oder Aminoketon abgeleitet ist;
X eine Gruppe ohne lichtempfindliche Gruppe ist;
n einen Wert von mindestens 1 bis m hat und m der Wertigkeit des Metalls entspricht.

Mit Vorteil enthält die Zusammensetzung über 5 Gew.-%, bevorzugt 5 bis 90 Gew.-%, besonders bevorzugt 30 bis 90 Gew.-% des ein- oder mehrkernigen Metallkomplexes.

Die Zusammensetzung kann noch zusätzlich ein oder mehrere Lösungsmittel enthalten. Als Lösungsmittel eignen sich alle Lösungsmittel, in welchen sich das organische Polymer und der Metallkomplex löst. Der Anteil an Lösungsmittel kann an die Erfordernisse angepasst
werden und kann zwischen 0 und 60 Gew.-% betragen. Bevorzugt sind organische Lösungsmittel. Es können auch Mischungen eingesetzt werden. Beispiele für organische Lösungsmittel sind Ketone, wie Aceton, Ester, wie Ethylacetat, Ether, wie Diethylether oder Tetrahydrofuran, Glycole, wie Ethylenglykol, Aliphatische, aromatische oder halogenierte Kohlenwasserstoffe, wie Hexan, Benzol, Dichlormethan oder Chloroform.

Außerdem können noch im geringen Maße, bis zu 5 Gew.-%, übliche Additive, wie Netzhilfsmittel, Haftvermittler, Verlaufsmittel, Antioxidationsmittel, Stabilisatoren, Farbstoffe, photochrome oder thermochrome Verbindungen oder Weichmacher enthalten sein.

Ohne an eine bestimmte Theorie gebunden zu sein, wird davon ausgegangen, dass sich der Metallkomplex entsprechend seiner lichtempfindlichen Gruppen zersetzt und auf diese Weise zur lokalen Entstehung von anorganischen Komponenten in der Zusammensetzung führt. Dadurch wird eine Potentialdifferenz erzeugt, welche die Diffusion weiterer Metallkomplexe zu dieser Stelle begünstigt, welche ebenfalls zersetzt werden. Dadurch kommt es zu einer Änderung der lokalen Brechzahl am Ort der Zersetzung und zum Aufbau eines Brechzahlgradienten.

Der Metallkomplex enthält ein Metall der 4. bis 12. Gruppe, nämlich Ti, Zr, Ta, V, Nb, Cr, Mo, W, Mn oder Re, besonders bevorzugt Ti, Ta oder Zr.

Unter lichtempfindlichen Gruppen werden im Sinne der Erfindung Gruppen verstanden, welche sich bei der Einwirkung von Licht und/oder Temperatur zersetzen. Dies bedeutet insbesondere die Spaltung von kovalenten Bindungen. Dabei kommt es nicht zu einer Polymerisation oder Polykondensation dieser Gruppen. Erst durch die Zersetzung dieser Gruppen wird der Komplex destabilisiert.

Neben den lichtempfindlichen Gruppen kann der Komplex noch weitere Gruppen enthalten, welche den Komplex stabilisieren, beispielsweise Alkoxide, 1,3-Diketone, Amine, Amide, oder Cyclopentadiene.

In einer vorteilhaften Weiterbildung enthält der ein- oder mehrkernige Metallkomplex ausschließlich lichtempfindliche Gruppen.

Lichtempfindliche Gruppen sind Gruppen, welche Norrish-Typ I-Reaktionen durchführen können. Dies sind beispielsweise übliche Photoinitiatoren für Polymerisationen, wie α-Hydroxyketone, Glyoxylate oder Aminoketone. Beispiele für kommerziell Gruppen sind Photoinitiatoren vom Irgacure^{®} oder Darocur^{®}-Typ, wie Irgacure 184 (1-Hydroxy-cyclohexyl-phenyl-ketone), Irgacure 2959 (2-Hydroxy-1-[4-(2-hydroxyethoxy)phenyl]-2-methyl-1-propanon), Irgacure 754, Irgacure 651 (α,α-dimethoxy-α-phenylacetophenon), Irgacure 819, Darocur 1173 (2-Hydroxy-2-methyl-1-phenyl-1-propanon), Darocur MBF (Methylbenzoylformat), Darocur 4265, Darocur TPO. Es können auch Derivate dieser Photoinitiatoren verwendet werden. Beispielsweise Derivate, welche die Anbindung an das Metallzentrum ermöglichen, Beispielsweise durch zusätzlich eingeführte Gruppen, wie Hydroxylgruppen, C₁₋₂-Alkoxygruppen, Karbonsäuregruppen oder Amine.

In einer vorteilhaften Weiterbildung der ist der reaktive Bereich der lichtempfindlichen Gruppe maximal über drei Bindungen mit dem Metall des Metallkomplexes verbunden. Dabei stellt der reaktive Bereich der Gruppe, den Bereich der Struktur dar, welcher maßgeblich an der Reaktion der Gruppe mit Licht beteiligt ist. Im Falle von α-Hydroxyketonen wäre dies der die Kombination aus der Hydroxylgruppe und der dazu benachbarten Ketogruppe. Bevorzugt ist eine möglichst direkte Nachbarschaft zwischen dem Metallzentrum und dem reaktiven Bereich der Gruppe, beispielsweise durch die direkte Verbindung über die α-Hydroxylgruppe der α-Hydroxyketone, beispielsweise von Irgacure 184 oder Darocur 1173. Solche Verbindungen können beispielsweise durch Umsetzung der entsprechenden Metallchloride oder Metallalkoxide erhalten werden.

Das Polymer der Zusammensetzung kann ein organisches Polymer sein. Bevorzugt ist es in der Zusammensetzung löslich. Das Polymer nimmt bevorzugt nicht an der Bildung des Brechzahlgradienten teil, sondern dient als Matrix für den sich bildenden Brechzahlengradient. Allerdings kann der Gehalt an Polymer in der Zusammensetzung die Bildung des Brechzahlgradienten beeinflussen, beispielsweise durch Beeinflussung der Diffusion. Außerdem dient das Polymer nach der Herstellung des Brechzahlengradienten der Stabilisierung dieses Gradienten.

Der Anteil an Polymer kann zwischen 1 und 99 Gew.-% liegen. Bevorzugt ist ein Gehalt von 30 bis 80 Gew.-%.

Das Polymer weist in einer vorteilhaften Weiterbildung keine reaktiven Gruppen auf, welche durch die Zersetzung des Metallkomplexes polymerisieren oder polykondensieren können.

Bevorzugt ist ein Polymer mit einem mittleren Molekulargewicht von über 50.000 Dalton, bevorzugt über 150.000 Dalton.

Das Polymer kann ein polymerisiertes oder polykondensiertes organisches Oligomer und / oder Prepolymer, ein organisches Polymer und / oder ein Kondensat aus einer oder mehreren hydrolysierbaren, gegebenenfalls organisch modifizierten anorganischen Verbindungen umfassen. Bevorzugt ist ein organisches Polymer. Bei den organischen Polymeren kann es sich um beliebige bekannte Kunststoffe handeln. Bevorzugt sind Polymere, die sich in den oben genannten Lösungsmitteln oder Gemischen davon lösen, z.B. Polyacrylsäure, Polymethacrylsäure und Derivate, Polyacrylate, Polymethacrylate, Polyethylenglykole, Polyolefine, Polystyrol und Polystyrol-Derivate, Polyamide, Polyimide, Polyvinyl-Verbindungen wie Polyvinylchlorid, Polyvinylalkohol, Polyvinylbutyral, Polyvinylacetat, Polyvinylpyrrolidon, Paravinylguajacol und entsprechende Copolymere, z.B. Poly-(ethylenvinylacetat), Polyester, z.B. Polyethylenterephthalat oder Polydiallylphthalat, Polyarylate, Polycarbonate, Polyether, z.B. Polyoxymethylen, Polyethylenoxid und Polyphenylenoxid, Polyetherketone, Polysulfone, Polyepoxide, Fluorpolymere, z. B. Polytetrafluorethylen, und Organopolysiloxane. Vorzugsweise handelt es sich um transparente Polymere. Bevorzugt sind Poly(meth)acrylsäure und Derivate, Poly(meth)acrylate, Poly(meth)acrylnitrile, Polystyrole oder Polystyrol-Derivate, Polyalkene, halogenierte Polyalkene, Polyvinylacetat, Polyvinylpyrrolidon, Polyvinylcarbazol, Poly(polyethylenglycol)-(meth)acrylate, Poly(polyethylenglycol)di(meth)acrylate. Die Zusammensetzung kann als Beschichtung oder als Formmasse verwendet werden. Der Gehalt an Lösungsmittel kann beim Aufbringen kann sich vom Gehalt während der Behandlung zur Erzeugung des Brechzahlgradienten unterscheiden.

Die Zusammensetzung kann auch Sensibilisatoren, wie beispielsweise Benzophenone enthalten, um beispielsweise eine Sensitivität der Zusammensetzung für eine bestimmte Wellenlänge zu erreichen. Der Anteil dieser Sensibilisatoren kann bei 0 bis 15 Gew.-% liegen.

Die Zusammensetzung kann auch noch weitere Komponenten, beispielsweise hoch- oder niedrigbrechende Nanopartikel, beispielsweise aus Metalloxiden enthalten, welche auch oberflächenmodifiziert sein können.

Die Zusammensetzung kann auch noch ein weiteres härtbares anorganisches oder organisches Matrixmaterial enthalten. Dieses Matrixmaterial kann nach der Herstellung des Brechzahlengradienten zur weiteren Stabilisierung des Gradienten gehärtet werden. Mit Vorteil ist dieses Material inert gegenüber den Reaktionen, welche zur Herstellung des Brechzahlengradienten führen.

Der Metallkomplex besitzt die Formel

X_{(m- n)}MR¹ₙ (I)

wobei M Ti, Zr, Ta, V, Nb, Cr, Mo, W, Mn oder Re ist.

R¹ ist eine lichtempfindliche Gruppe und X eine Gruppe ohne lichtempfindliche Gruppe. Der Wert von m ist größer als n und m entspricht der Wertigkeit des Metalls. Dabei ist n mindestens 1 und bevorzugt gleich m.

In einer bevorzugten Weiterbildung ist der reaktive Bereich der lichtempfindlichen Gruppe, welcher wie vorstehend definiert ist, maximal über drei Bindungen mit M verbunden. Dies bedeutet beispielsweise, dass in einer Strukturformel des Komplexes entsprechend der üblichen Valenzschreibweise, nicht mehr als drei Bindungen zwischen dem reaktiven Bereich und dem Metallkomplex liegen. Der reaktive Bereich sind dabei alle Bindungen, welche direkt an der photochemischen Reaktion der lichtempfindlichen Gruppe beteiligt sind, beispielsweise mindestens die gespaltene Bindung im Falle einer Norrish-Typ-I-Reaktion.

R¹ ist von üblichen Photoinitiatoren für Polymerisationen, wie α-Hydroxyketonen, Glyoxylaten oder Aminoketonen abgeleitet, beispielsweise von Photoinitiatoren vom Irgacure^{®} oder Darocur^{®}-Typ, wie Irgacure 184 (1-Hydroxy-cyclohexyl-phenyl-ketone), Irgacure 2959 (2-Hydroxy-1-[4-(2-hydroxyethoxy)phenyl]-2-methyl-1-propanon) Irgacure 754, Irgacure 651 (α,α-dimethoxy-α-phenylacetophenon), Irgacure 819, Darocur 1173 (2-Hydroxy-2-methyl-1-phenyl-1-propanon), Darocur MBF (Mehtylbenzoylformat), Darocur 4265, Darocur TPO. Dabei können diese Strukturen über die vorhandenen oder über eingeführte Heteroatome oder kurze Alkoxybrücken an M gebunden sein. Bevorzugt ist eine Anbindung über Heteroatome des reaktiven Bereichs der Photoinitiatoren, beispielsweise über die α-Hydroxylgruppe der α-Hydroxyketone. Bevorzugt enthält der Metallkomplex nur eine Art von Ligand.

X ist eine Gruppe, welche keine lichtempfindliche Gruppe enthält. Bevorzugt sind Alkoxide, 1,3-Diketone, Amine, welche auch alkyliert sein können, Amide, oder Cyclopentadiene. Möglich sind auch mehrzähnige Liganden, welche ebenfalls Heteroatome enthalten können.

Bevorzugt ist es ein Metallkomplex der Formel

M-(-Z-CR²R³-CO-R⁴)ₘ (II)

Wobei M wie vorstehend definiert ist. Z ist O oder N, wobei Z im Falle von N auch alkyliert, bevorzugt methyliert sein kann.

R² und R³ sind gleich oder verschieden, bevorzugt ein C₁-C₁₂ Alkylrest oder ein Arylrest, besonders bevorzugt Methyl oder Ethyl, Propyl, Isopropyl oder Phenyl, wobei die Reste auch substituiert sein können. R² und R³ können auch über eine Alkylenbrücke miteinander verbunden sein, bevorzugt eine C₆-Alkylenbrücke, welche ebenfalls durch Heteroatome unterbrochen sein kann.

R⁴ ist eine C₁- bis C₆-Alkylgruppe oder unsubstituierte oder mit C₁ bis C₃ Alkylresten substituierte Arylgruppe, wobei die Gruppen auch Heteroatome oder Halogene enthalten können. Bevorzugt ist R⁴ eine unsubstituierte oder substituierte Phenylgruppe, wobei die Gruppe mit Halogenen, oder Methyl- oder Ethylgruppen substituiert sein kann. Bevorzugt ist R⁴ eine Phenylgruppe.

M entspricht der Wertigkeit des Metalls.

Beispiele für solche Komplexe sind Ti(OC₃H₆COC₆H₅)₄, Zr(OC₃H₆COC₆H₅)₄ oder Ta(OC₃H₆COC₆H₅)₄.

Des Weiteren betrifft die Erfindung auch ein Verfahren zur Herstellung eines optischen Elements.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen. In einem ersten Schritt wird eine Zusammensetzung aus mindestens einem organischen Polymer und mindestens einem Metallkomplex, welcher mindestens eine licht- und/oder temperaturempfindliche Gruppe, sowie mindestens einem Lösungsmittel hergestellt.

Es wird die erfindungsgemäße Zusammensetzung verwendet. Als Metallkomplex wird ein erfindungsgemäßer Metallkomplex wie für die Zusammensetzung eingesetzt.

Mit Vorteil liegt die Zusammensetzung als Lösung oder Emulsion vor.

Die Komponenten zur Herstellung der Zusammensetzung bzw. einer Vorstufe davon können in beliebiger Weise und Reihenfolge miteinander gemischt werden.

Es können auch noch von weitere Additive, wie beispielsweise Netzhilfsmittel, Haftvermittler, Verlaufsmittel, Antioxidationsmittel, Stabilisatoren, Farbstoffe, photochrome oder thermochrome Verbindungen oder Weichmacher, zugegeben werden, allerdings nur bis zu 5 Gew.-%.

Die Zusammensetzung kann als Beschichtungszusammensetzung oder als Formmasse verwendet werden. In Abhängigkeit davon kann die Viskosität beispielsweise durch die Menge an Lösungsmittel oder durch die Art des verwendeten Polymers angepasst werden. So wird die Zusammensetzung auf eine Oberfläche aufgebracht oder in eine Form eingebracht.

Gegebenenfalls kann vor der weiteren Behandlung der Gehalt an Lösungsmittel reduziert werden, beispielsweise durch Trocknung.

Im nächsten Schritt wird in der Zusammensetzung eine Potentialdifferenz zur gerichteten Diffusion der Metallkomplexe durch lokale Zersetzung der Metallkomplexe erzeugt.

Bei der Potentialdifferenz wird vorzugsweise eine chemische Potentialdifferenz erzeugt, beispielsweise analog dem vorstehend beschriebenen Colburn-Haines-Effekt. Bei einer lokalen (z. B. thermisch und/oder photochemisch induzierten) Zersetzung des Metallkomplexes führt dies zu einer Verringerung der Konzentration des Metallkomplexes in diesen Bereichen. Dies führt zu einer gerichteten Diffusion von unzersetzten Metallkomplexen in die (erwärmten bzw. belichteten) Bereiche, um die chemische Potentialdifferenz auszugleichen. Diese Metallkomplexe stehen in diesen Bereichen zur Zersetzung zur Verfügung. Dies führt in den erwärmten bzw. belichteten Bereichen zu einer Veränderung der optischen Dichte und somit zu einer lokalen Erhöhung oder Erniedrigung der Brechzahl.

Dabei wird unter Zersetzung die Umwandlung des Metallkomplexes unter Änderung der Brechzahl verstanden. Bevorzugt zersetzt sich der Komplex unter Spaltung der licht- und/oder temperaturempfindlichen Liganden. Ohne an eine Theorie gebunden zu sein, entstehen dabei anorganische Verbindungen, beispielsweise Metalloxide, welche die lokale Brechzahl beeinflussen. Dabei kommt es bevorzugt zu keiner Polymerisations- oder Polykondensationsreaktion der Liganden, wie beispielsweise bei der Bildung von Polymeren. Die Liganden wirken auch nicht als Polymerisationsstarter. Daher enthält die Zusammensetzung bevorzugt keine Monomere für Polymere, auch nicht als Liganden, wie beispielsweise Methacrylate oder Epoxide. Die Veränderung der Brechzahl geht von der bei der Zersetzung gebildeten anorganischen Komponente des Komplexes aus.

Die chemische Potentialdifferenz wird vorzugsweise durch Belichtung oder Elektronenbestrahlung, insbesondere durch holographische oder lithographische Techniken oder über die Maskaligner-Technik, erzeugt. Durch selektive Bestrahlung oder Belichtung der Zusammensetzung kann z. B. an lokalen Stellen gezielt eine Zersetzung des Metallkomplexes ausgelöst werden, die zu einer chemischen Potentialdifferenz führt, die wiederum zur gerichteten Diffusion der Metallkomplexe und zur Ausbildung eines Brechzahlgradienten führt.

Ohne an ein bestimmtes System gebunden zu sein, ließe es sich auch so erklären, dass durch die lokale Zersetzung eine Komponente mit einer bestimmten Brechzahl gebildet wird, bzw. ihre Konzentration dort erhöht wird, während im Gegenzug die Brechzahl der anderen Bereiche durch die Diffusion der Metallkomplexe ebenfalls verändert wird.

Daraus folgt, dass, wie vorstehend erwähnt, die Veränderung der Brechzahl immer in Relation zu den benachbarten Bereichen zu sehen ist. Entscheidend ist der daraus resultierende Unterschied in der Brechzahl. Welcher Bereich eine höhere, bzw. niedrigere Brechzahl aufweist, kann beispielsweise durch die Wahl des Polymers oder der Metallkomplexe, bzw. des Metalls, oder anderer Komponenten bestimmt werden.

Für die Belichtungsverfahren werden bevorzugt UV-Licht oder Laserlicht eingesetzt. Bei Verwendung eines Lasers als Lichtquelle können über holographische Techniken sowohl periodische Gitterstrukturen als auch Fresnelstrukturen hergestellt werden. Die durch Interferenz auftretenden Intensitätsprofile wirken als Polymerisationssenken. Für die besonders bevorzugte holographische Belichtung können z. B. mittels Zwei-Wellenmischen phasenmodulierte Volumenhologramme als Transmissions- oder Reflexionshologramme hergestellt werden.

Als kohärente Lichtquelle kann z. B. ein Argon-Ionen-Laser dienen.

Nach der Herstellung der Gradientenstruktur können die nicht zersetzten Metallkomplexe durch eine nicht-lokale Zersetzung, beispielsweise eine nicht intensitätsmodulierte Belichtung, gleichmäßig abreagieren. Da dabei keinerlei gerichtete Diffusion mehr auftritt, kommt es maximal zu einer Abschwächung des bereits erzeugten Brechzahlgradienten. Allerdings kann dadurch die Haltbarkeit der hergestellten Gradientenstrukturen deutlich verbessert werden.

Des Weiteren betrifft die Erfindung ein optisches Element erhältlich aus einer erfindungsgemäßen Zusammensetzung.

Das erfindungsgemäße optische Elemente oder die Zusammensetzungen eignen sich insbesondere zur Herstellung von optischen Elementen mit einem Brechzahlgradienten. Die optischen Elemente sind insbesondere als holographische Anwendungen, Lichtmanagementfolien, Diffusoren, planare Gradientenindexlinsen in der abbildenden Optik, Head-up-Displays, Head-down-Displays, Lichtwellenleiter, vor allem in der optischen Nachrichten- und Übertragungstechnik, und optische Datenspeicher geeignet. Beispiele für herstellbare optische Elemente sind Sicherheitshologramme, Bildhologramme, digitale Hologramme zur Informationsspeicherung, Systeme mit Komponenten, die Lichtwellenfronten verarbeiten, planare Wellenleiter, Strahlteiler und Linsen.

Außerdem betrifft die Erfindung die Verwendung von erfindungsgemäßen optischen Elementen für holographische Anwendungen, planare Gradientenindexlinsen in der abbildenden Optik, Lichtmanagementfolien, Diffusoren, Head-up-Displays, Head-down-Displays, Lichtwellenleiter und optische Datenspeicher.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt.

So umfassen beispielsweise Bereichsangaben stets alle - nicht genannten - Zwischenwerte und alle denkbaren Teilintervalle.

Zuerst wird eine Zusammensetzung aus dem Metallkomplex und dem Polymer hergestellt. Um eine homogene Zusammensetzung zu erhalten, wird bevorzugt eine Lösung hergestellt. Dabei können die Komponenten in unterschiedlicher Reihenfolge vermischt werden. Bevorzugt enthält diese Mischung einen Anteil von 5 bis 60 Gew.-% Metallkomplex, 5 bis 50 Gew.-% Polymer und 20 bis 80 Gew.-% eines oder mehrerer Lösungsmittel.

Für die Beschichtung werden vorzugsweise für optische Anwendungen geeignete Substrate ausgewählt, wie beispielsweise Glas, Keramik, Silizium, Metall, Halbleiter-Materialien oder (vorzugsweise transparente) Kunststoffe, wie PET, PE und PP. Ein besonders bevorzugtes Substrat ist eine Kunststofffolie. Die Beschichtung kann nach üblichen Methoden erfolgen, z. B. durch Tauchen, Fluten, Rakeln, Gießen, Schleudern, Spritzen, Aufstreichen, Slot-Coating, Meniskus-Coating, Foliengießen, Spinnen oder Sprühen. Hierfür eignen sich natürlich flüssige Vorstufen der Zusammensetzung, wobei die erforderliche Viskosität beispielsweise durch Zugabe oder Entfernen von Lösungsmittel(n) eingestellt werden kann. Alternativ kann die Wahl des Polymers die Viskosität beeinflussen. Bevorzugte Schichtdicken (der fertigen Beschichtung) liegen bei 0.2 bis 300 µm, besonders bevorzugt zwischen 0.2 bis 100 µm.

In dieser Form kann das Folienmaterial aufgewickelt, lichtgeschützt und klimatisiert (15 bis 30 °C) zwischengelagert werden. Auf diese Weise kann auch ein Folienverbund bzw. -komposit hergestellt werden. Folien mit einer Beschichtung, die einen Brechzahlgradienten aufweist, auf die gegebenenfalls eine zweite Folie auflaminiert wurde (Folienkomposit) sind bevorzugte erfindungsgemäße optische Elemente.

Anschließend wird in der Zusammensetzung auf die oben beschriebene Weise eine Potentialdifferenz erzeugt, so dass durch gerichtete Diffusion und der induzierten lokalen Zersetzung des Metallkomplexes ein Brechzahlgradient gebildet wird. Die Potentialdifferenz wird bevorzugt durch ein Belichtungsverfahren erzeugt.

In einer bevorzugten Ausführungsform der Erfindung zur Herstellung eines Transmissionshologramms wird eine solche erfindungsgemäße Zusammensetzung auf eine Glasoberfläche aufgetragen und getrocknet. Dabei wurden Schichten mit einer Dicke zwischen 8 und 200 µm hergestellt. Mit Hilfe von Zwei-Wellenmischen eines Laserstrahls mit einer Wellenlänge zwischen 300 nm und 500 nm und einer Intensität zwischen 7 und 300 mW/cm² werden phasenmodulierte Volumenhologramme sowohl als Transmissions- als auch als Reflexionshologramme erzeugt. Die Belichtungszeit beträgt zwischen 1 Sekunden und 10 Minuten, bevorzugt zwischen 1 und 3 Minuten.

Die sich bei Erzeugung einer Potentialdifferenz abspielenden Vorgänge werden nachstehend für eine bevorzugte Ausführungsform erläutert.

Durch eine lokale Belichtung werden die Metallkomplexe lokal unter Veränderung des lokalen Brechungsindex zersetzt. Dadurch wird ein chemischer Potentialgradient für noch nicht zersetzte Metallkomplexen zu der unbelichteten Nachbarregion gebildet. Aus dieser Nachbarregion diffundieren daher weitere Metallkomplexe in die belichtete Region. Dieser Prozess kann während und nach der Belichtung erfolgen und dauert zwischen wenigen Sekunden bis wenige Minuten, je nach Belichtungsbedingungen und Temperatur. Durch den Brechzahlunterschied zwischen den verschiedenen Bereichen mit unterschiedlicher Zersetzung entsteht auf diese Weise ein lokaler Brechzahlgradient.

Zusammengefasst erlaubt das erfindungsgemäße Verfahren auf eine kostengünstige und effiziente Weise die Herstellung von Gradientenstrukturen, welche anorganische Komponenten enthalten, ohne auf die schnelle Diffusion der aktiven Komponenten verzichten zu müssen. Dadurch kann beispielsweise bei gleicher Dicke des Materials eine wesentliche Verbesserung der optischen Eigenschaften erreicht werden. Die Produktion solcher Systeme wird auf diese Weise deutlich einfacher und kostengünstiger. Gleichzeitig ist auch eine große Variabilität beispielsweise bezüglich der verwendeten Metallkomplexe und Polymere realisierbar, da die Eigenschaften der Metallkomplexe und/oder des Polymers sehr flexibel an die gewünschten Gegebenheiten angepasst werden können.
- Fig. 1: Experimenteller Aufbau zur Erzeugung und Untersuchung der Hologramme
- Fig. 2: Verlauf des Beugungswirkungsgrades am ersten Beugungsmaximum aus Beispiel 1;
- Fig. 3: Verlauf des Beugungswirkungsgrades am ersten Beugungsmaximum aus Beispiel 2;
- Fig. 4: Lichtmikroskopische Aufnahme des Hologramms aus Beispiel 2;
- Fig. 5: Verlauf des Beugungswirkungsgrades am ersten Beugungsmaximum des Hologramms aus Beispiel 3; und
- Fig. 6: Verlauf des Beugungswirkungsgrades am ersten Beugungsmaximum des Hologramms aus Beispiel 4.

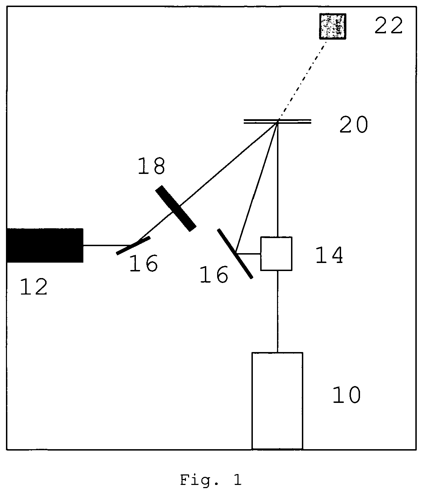

Fig. 1 zeigt den verwendeten Aufbau zur Erzeugung der Hologramme. Zum Schreiben wurde ein Ar⁺-Ionenlaser (10) verwendet. Der Schreibprozess kann durch einen He-Laser (12) kontrolliert werden. Das Hologramm selbst wurde mittels eines Strahlteilers (14) im Strahlengang des Ar⁺-Ionenlasers (10) erzeugt. Diese Strahlen und der Strahl des He-Lasers (12) über mehrere Spiegel (16) auf die Probe (20) geleitet. Zur Detektion des Hologramms mit Hilfe des He-Lasers (12) wurde ein Chopper (18) und ein Detektor mit Anschluss an einen Lock In Amplifier (22) verwendet.

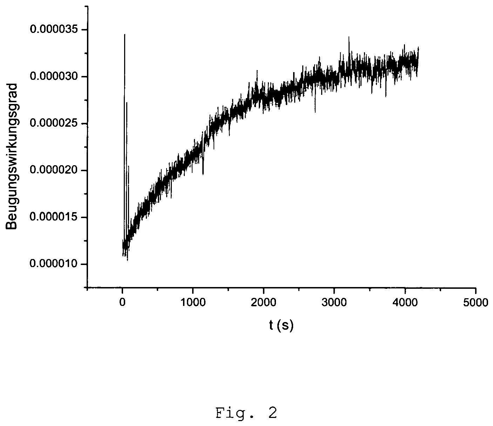

Fig. 2 zeigt den Verlauf des Beugungswirkungsgrades am ersten Beugungsmaximum nach dreimaligem kurzzeitigem Bestrahlen einer Zusammensetzung aus Beispiel 1;

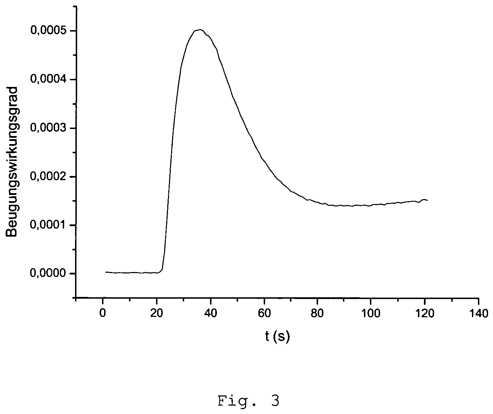

Fig. 3 zeigt den Verlauf des Beugungswirkungsgrades am ersten Beugungsmaximum aus Beispiel 2. Bei der Belichtung war die Probe nicht mit einer Glasplatte abgedeckt.

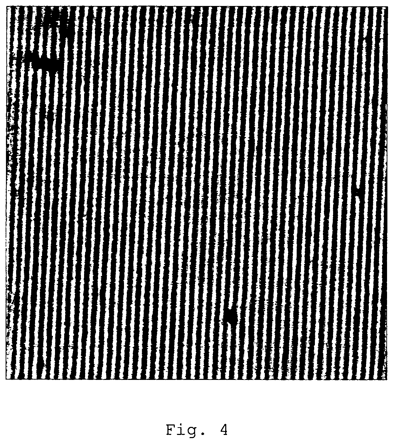

Fig. 4 zeigt das erhaltene Hologramm aus Beispiel 2 unter einem Lichtmikroskop. Der Abstand zwischen den Linien beträgt von Mitte zu Mitte ∼10 µm, die Breite der Linien beträgt etwa 2 µm.

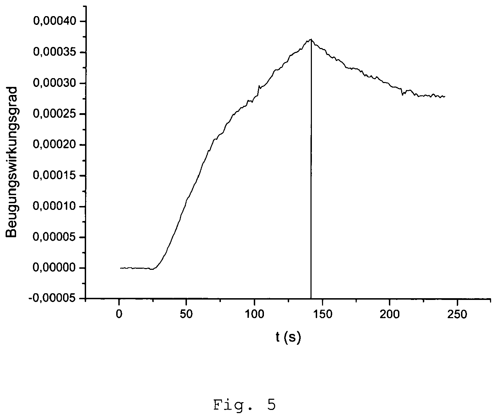

Fig. 5 Verlauf des Beugungswirkungsgrades am ersten Beugungsmaximum für das Hologramm aus Beispiel 3;

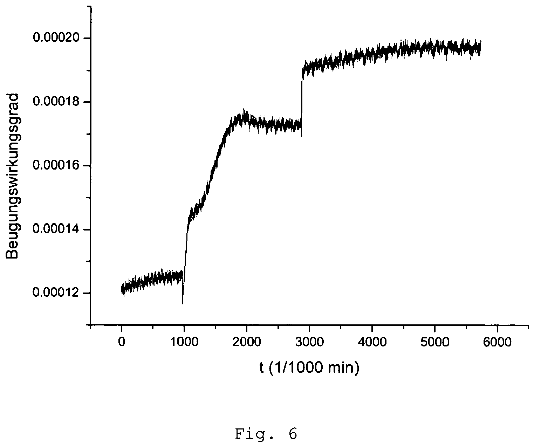

Fig. 6 Verlauf des Beugungswirkungsgrades am ersten Beugungsmaximum für das Hologramm aus Beispiel 4. Es war eine sehr schwache Beugung erkennbar, die allerdings stabil blieb und auch nach zwei Tagen noch mit Hilfe eines Lasers erkennbar war.

Es sind zahlreiche Abwandlungen und Weiterbildungen der beschriebenen Ausführungsbeispiele verwirklichbar.

### Material und Methoden

Die Synthese der Metallalkoxide wurde in einer abgedunkelten modifizierten Stockschen Hochvakuumapparatur mit getrocknetem Stickstoff als Schutzgas durchgeführt. Die verwendeten Lösungsmittel wurden nach Standardverfahren getrocknet und gelagert. Das verwendete Titantetraisopropanolat wurde vor Gebrauch destilliert, das verwendete Zirkoniumpropanolat in einer Kugelrohrdestille sublimiert und der verwendete Photostarter über Molekularsieb getrocknet.

Die ¹H- und die ¹³C-NMR-Spektren wurden an einem NMR-Spektrometer AC 200 F der Firma Bruker in den angegebenen Lösungsmitteln mit 5 - 10 Volumenprozent Deuterochloroform CDCl₃ aufgenommen. Die CHN-Analyse wurde mittels Verbrennungsanalyse auf einem CHN-900 Elemental Analysator der Firma Leco Corporation durchgeführt.

### Synthese von Ti(OC₃H₆COC₆H₅)₄ (1)

2.73 g (9.58 mmol) gereinigtes Titantetraisopropanolat werden bei Raumtemperatur in 20 ml wasserfreiem Tetrahydrofuran vorgelegt. Zu dieser Lösung werden langsam 6.16 ml (38,32 mmol) 2-Hydroxy-2-methyl-1-phenyl-1-propanon (Darocur 1173; Ciba Specialty Chemicals) in 20 ml wasserfreiem Tetrahydrofuran zugetropft. Nach 12 Stunden Rühren wird die Reaktionslösung eingeengt, sie färbt sich dabei dunkel orange ein. Aus dieser Lösung kristallisieren unter weiterhin reduziertem Druck im Verlauf von 3 Tagen blassgelbe Kristalle von Ti(OC₃H₆COC₆H₅)₄ (1) aus. Die überstehende Lösung wird abgetrennt und die Kristalle werden mit Tetrahydrofuran gewaschen bzw. daraus umkristallisiert.

¹H-NMR (THF; CDCl₃; ppm) : 1.46 (s; 24H; CH₃), 8.16 - 8.20 (dd; 8H; aromatische H), 7.30 - 7.40 (m; 12H; aromatische H), ¹³C-NMR (THF; CDCl₃ ; ppm): 26.76 (8C), 75.78 (4C), 126.67 (8C), 128.95 (8C), 130.87 (4C), 134.12 (4C), 202.41 (4C); Elementaranalyse: berechnet: C 68.59 %, H 6.28 %; gefunden: C 68.69 %, H 6.07 %.

Analog wurden die Synthesen von Zr(OC₃H₆COC₆H₅)₄ (2) und Ta(OC₃H₆COC₆H₅)₅ (3) durchgeführt.

### Herstellung von Beschichtungen

Die Sole wurden durch Lösen der Metallkomplexe in Tetrahydrofuran bzw. Aceton sowie anschließendem Mischen mit einer Polyvinylacetat-Lösung (*Synthomer M50*) in THF bzw. Aceton hergestellt.

Zur Herstellung der Filme wurden auf einem Glasobjektträger (*Marienfeld*) mehrere Tropfen der jeweiligen Sole aufgebracht anschließend wurden Aluminiumfolienstreifen mit einer Dicke von ∼ 10 - 12 µm als Abstandhalter aufgelegt und die Proben mit einem weiteren Objektträger abgedeckt. Weiterhin wurden Filme ohne zweiten Objektträger hergestellt und untersucht.

Die Holographieversuche durch Zwei-Wellenmischen mit einem Ar⁺-Ionen-Lasers (λ = 351 nm) Model 2000 der Firma *Spectra Physics* durchgeführt und der Schreibprozess in Echtzeittransmissionsmessung mit Hilfe eines Helium-Lasers (λ = 632,9 nm) verfolgt (Siehe Fig. 1). Das Signal des He-Lasers wurde mittels eines Choppers mit einer Frequenz von 124 Hz moduliert und durch einen Detektor, der an einen Lock In Amplifier (Stanford Research Systems SR 850 DSP) angeschlossen war, aufgezeichnet.

### Beispiel 1: Belichtung von 1

0.45 g von 1 in 4,00 g einer Polyvinylacetat-Lösung (10 g Polyvinylacetat, *Synthomer M50*, in 30 ml THF) . Es wurde 3x für je 2 Sekunden belichtet (P = 6,5 mW), danach bildete sich das Hologramm, welches allerdings nur mit Hilfe des He/Ne-Lasers zu erkennen war (Fig. 2).

### Beispiel 2: Belichtung von 1 ohne zusätzlichen Objektträger als Abdeckung

Es wurde eine Lösung von 1 und einer Polyvinylacetat-Lösung (10 g auf 30 ml THF) im Verhältnis 1:2 bezogen auf Polyvinylacetat auf einem Objektträger verteilt und trocknen lassen. Dort, wo die Schicht sehr dick war, wurde sie weißlich, sonst war sie klar. Danach wurde ein Hologramm aufgenommen, mit 13,0 mW Leistung. Nach 20 s ruhen wurde für 20 s belichtet und anschließend der weitere Verlauf für weitere 80 s verfolgt (Fig. 3,4).

Die Schicht wurde mit einer Rakel appliziert. Die Nassfilmdicke betrug 120 µm. Der Beugungswirkungsgrad der mit einer Rakel hergestellten Schicht betrug 1,4 %.

### Beispiel 3: Belichtung von 2 ohne zusätzlichen Objektträger als Abdeckung

Es wurde eine Lösung von 2 und einer Polyvinylacetat-Lösung (10 g auf 30 ml THF) im Verhältnis 1:1 auf einem Objektträger verteilt und trocknen gelassen. Dabei verfärbte sich die Schicht recht schnell komplett weißlich. Dennoch war es möglich ein Hologramm zu erzeugen (Leistung: 13,0 mW).

Es wurde zuerst für 20 s ruhen gelassen und anschließend für 2 Min. belichtet, danach wurde der Kurvenverlauf für 100 s verfolgt (Fig 5).

### Beispiel 4: Belichtung von 3

7.73 g 3 wird mit Polyvinylacetat im Verhältnis 1:1 gemischt und mit 25 ml Aceton gemischt. Aus der weißen Mischung setzt sich ungelöstes 3 ab. Mit der überstehenden klaren Lösung werden Holographieexperimente durchgeführt. Dabei wurde die Schicht für 1 min ruhen lassen und anschließend für 3 min belichtet. Danach wurde für weitere ∼2.5 min der Verlauf des Beugungswirkungsgrades am ersten Beugungsmaximum verfolgt (Fig. 6).

### Bezugszeichen

- 10: Ar⁺-Ionenlaser
- 12: He-Laser
- 14: Strahlteiler
- 16: Spiegel
- 18: Chopper
- 20: Probe
- 22: Detektor mit Anschluss an einen Lock in Amplifier

### Liste der zitierten Literatur:

US 5,552,261
US 5,529,473
US 2005/0101698 A1

## Patentansprüche

1. Zusammensetzung zur Herstellung von optischen Elementen mit einem Brechzahlgradienten, **dadurch gekennzeichnet, dass**
die Zusammensetzung
a) mindestens ein organisches Polymer; und
b) mindestens einen Metallkomplex der Formel
X₍ₘ₋ₙ₎MR¹ₙ (I)
ist,
wobei M für Ti, Zr, Ta, V, Nb, Cr, Mo, W, Mn oder Re steht;
R¹ von einem α-Hydroxyketon, Glyoxylat oder Aminoketon abgeleitet ist;
X eine Gruppe ohne lichtempfindliche Gruppe ist;
n einen Wert von mindestens 1 bis m hat und m der Wertigkeit des Metalls entspricht.

2. Zusammensetzung nach Anspruch 1, **dadurch gekennzeichnet, dass**
der Metallkomplex sich unter Änderung der lokalen Brechzahl zersetzt.

3. Zusammensetzung nach einem der Ansprüche 1 oder 2, **dadurch gekennzeichnet, dass**
die Zusammensetzung keine Monomere für Polymere enthält.

4. Zusammensetzung nach einem der vorhergehenden Ansprüche, **dadurch gekennzeichnet, dass**
der ein- oder mehrkernige Metallkomplex ausschließlich lichtempfindliche Gruppen enthält.

5. Zusammensetzung nach Anspruch 4, **dadurch gekennzeichnet, dass**
der reaktive Bereich der lichtempfindlichen Gruppe maximal über drei Bindungen mit dem Metall des Metallkomplexes verbunden ist.

6. Zusammensetzung nach einem der vorhergehenden Ansprüche, **dadurch gekennzeichnet, dass**
das Polymer ein organisches Polymer ausgewählt aus der Gruppe Poly(meth)acrylsäure und Derivate, Poly(meth)acrylate, Poly(meth)acrylnitrile, Polystyrole oder Polystyrol-Derivate, Polyalkene, halogenierte Polyalkene, Polyvinylacetat, Polyvinylpyrrolidon, Polyvinylcarbazol, Poly(polyethylenglycol)-(meth)acrylate, Poly(polyethylenglycol)di(meth)acrylate ist.

7. Zusammensetzung nach einem der vorhergehenden Ansprüche, **dadurch gekennzeichnet, dass**
die Zusammensetzung ein oder mehrere Lösungsmittel enthält.

8. Zusammensetzung nach einem der vorhergehenden Ansprüche, **dadurch gekennzeichnet, dass**
der Metallkomplex ein Metallkomplex der Formel
M-(-Z-CR²R³-CO-R⁴)ₘ (II)
ist, wobei M wie vorstehend definiert ist,
Z ist 0 oder N, wobei Z im Falle von N auch alkyliert sein kann;
R² und R³ sind gleich oder verschieden ein C₁-C₁₂ Alkylrest oder ein Arylrest, wobei die Reste auch substituiert sein können oder über eine Alkylenbrücke miteinander verbunden sein können;
R⁴ ist eine C₁- bis C₆-Alkylgruppe oder unsubstituierte oder mit C₁ bis C₃ Alkylresten substituierte Arylgruppe, wobei die Gruppen auch Heteroatome oder Halogene enthalten können; und
m der Wertigkeit des Metalls entspricht.

9. Verfahren zur Herstellung eines optischen Elements enthaltend folgende Schritte:
a) Herstellen einer Zusammensetzung enthaltend
a1) mindestens ein organisches Polymer; und
a2) mindestens einen Metallkomplex der Formel
X₍ₘ₋ₙ₎MR¹ₙ (I)
ist,
wobei M für Ti, Zr, Ta, V, Nb, Cr, Mo, W, Mn oder Re steht;
R¹ von einem α-Hydroxyketon, Glyoxylat oder Aminoketon abgeleitet ist;
X eine Gruppe ohne lichtempfindliche Gruppe ist;
n einen Wert von mindestens 1 bis m hat und m der Wertigkeit des Metalls entspricht; sowie
a3) mindestens ein Lösungsmittel;
b) Aufbringen der Zusammensetzung auf eine Oberfläche oder in eine Form;
c) Erzeugen einer Potentialdifferenz zur gerichteten Diffusion der Metallkomplexe durch lokale Zersetzung der Metallkomplexe.

10. Optisches Element erhältlich aus einer Zusammensetzung gemäß einem der Ansprüche 1 bis 8 oder eines Verfahrens gemäß Anspruch 9.

11. Verwendung des optischen Elements nach Anspruch 10 für holographische Anwendungen, planare Gradientenindexlinsen in der abbildenden Optik, Lichtmanagementfolien, Diffusoren, Head-up-Displays, Head-down-Displays, Lichtwellenleiter und optische Datenspeicher.

## Claims

1. Composition for producing optical elements with a refractive index gradient, **characterized in that** the composition is
a) at least one organic polymer; and
b) at least one metal complex of the formula
X₍ₘ₋ₙ₎MR¹ₙ (I)
where M is Ti, Zr, Ta, V, Nb, Cr, Mo, W, Mn or Re;
R¹ is derived from an α-hydroxy ketone, glyoxylate or amino ketone;
X is a group without a light-sensitive group;
n has a value of at least 1 to m and m corresponds to the valency of the metal.

2. Composition according to Claim 1, **characterized in that**
the metal complex decomposes to alter the local refractive index.

3. Composition according to either of Claims 1 and 2, **characterized in that**
the composition does not contain any monomers for polymers.

4. Composition according to any of the preceding claims, **characterized in that**
the mono- or polynuclear metal complex comprises exclusively light-sensitive groups.

5. Composition according to Claim 4, **characterized in that**
the reactive region of the light-sensitive group is bonded to the metal of the metal complex via not more than three bonds.

6. Composition according to any of the preceding claims, **characterized in that**
the polymer is an organic polymer selected from the group of poly(meth)acrylic acid and derivatives, poly(meth)acrylates, poly(meth)-acrylonitriles, polystyrenes or polystyrene derivatives, polyalkenes, halogenated polyalkenes, polyvinyl acetate, polyvinylpyrrolidone, polyvinylcarbazole, poly(polyethylene glycol) (meth)acrylates, poly(polyethylene glycol) di(meth)acrylates.

7. Composition according to any of the preceding claims, **characterized in that**
the composition comprises one or more solvents.

8. Composition according to any of the preceding claims, **characterized in that**
the metal complex is a metal complex of the formula
M- (-Z-CR²R³-CO-R⁴)ₘ (II)
where M is as defined above;
Z is O or N, where Z in the case of N may also be alkylated;
R² and R³ are the same or different and are a C₁-C₁₂ alkyl radical or an aryl radical, where the radicals may also be substituted or may be joined to one another via an alkylene bridge;
R⁴ is a C₁- to C₆-alkyl group or an aryl group which is unsubstituted or substituted by C₁ to C₃ alkyl radicals, where the groups may also contain heteroatoms or halogens; and
m corresponds to the valency of the metal.

9. Process for producing an optical element, comprising the following steps:
a) producing a composition comprising
a1) at least one organic polymer; and
a2) at least one metal complex of the formula
X₍ₘ₋ₙ₎MR¹ₙ (I)
where M is Ti, Zr, Ta, V, Nb, Cr, Mo, W, Mn or Re;
R¹ is derived from an α-hydroxy ketone, glyoxylate or amino ketone;
X is a group without a light-sensitive group;
n has a value of at least 1 to m and m corresponds to the valency of the metal; and
a3) at least one solvent;
b) applying the composition to a surface or to a mold;
c) generating a potential difference for directed diffusion of the metal complexes through local decomposition of the metal complexes.

10. Optical element obtainable from a composition according to any of Claims 1 to 8 or a process according to Claim 9.

11. Use of the optical element according to Claim 10 for holographic applications, planar gradient index lenses in imaging optics, light management films, diffusers, head-up displays, head-down displays, light waveguides and optical data stores.

## Revendications

1. Composition pour la fabrication d'éléments optiques ayant un gradient d'indice de réfraction, **caractérisée en ce que** la composition contient
a) au moins un polymère organique ; et
b) au moins un complexe métallique de formule
X₍ₘ₋ₙ₎MR¹ₙ (I)
dans laquelle M représente Ti, Zr, Ta, V, Nb, Cr, Mo, W, Mn ou Re ;
R¹ est dérivé d'une α-hydroxycétone, d'un glyoxylate ou d'une aminocétone ;
X est un groupe sans groupe sensible à la lumière ;
n a une valeur d'au moins 1 à m et m correspond à la valence du métal.

2. Composition selon la revendication 1, **caractérisée en ce que** le complexe métallique se décompose avec modification de l'indice de réfraction local.

3. Composition selon l'une quelconque des revendications 1 ou 2, **caractérisée en ce que** la composition ne contient pas de monomères pour des polymères.

4. Composition selon l'une quelconque des revendications précédentes, **caractérisée en ce que** le complexe métallique à un ou plusieurs noyaux contient exclusivement des groupes sensibles à la lumière.

5. Composition selon la revendication 4, **caractérisée en ce que** la zone réactive du groupe sensible à la lumière est reliée au plus par trois liaisons avec le métal du complexe métallique.

6. Composition selon l'une quelconque des revendications précédentes, **caractérisée en ce que** le polymère est un polymère organique choisi dans le groupe constitué par l'acide poly(méth)acrylique et ses dérivés, les poly(méth)acrylates, les poly(méth)acrylonitriles, les polystyrènes ou les dérivés de polystyrène, les polyalcènes, les polyalcènes halogénés, le polyacétate de vinyle, la polyvinylpyrrolidone, le polyvinylcarbazole, le poly(méth)acrylate de polyéthylène glycol, le polydi(méth)acrylate de polyéthylène glycol.

7. Composition selon l'une quelconque des revendications précédentes, **caractérisée en ce que** la composition contient un ou plusieurs solvants.

8. Composition selon l'une quelconque des revendications précédentes, **caractérisée en ce que** le complexe métallique est un complexe métallique de formule
M- (-Z-CR²R³-CO-R⁴)ₘ (II)
M étant tel que défini précédemment,
Z représentant O ou N, Z pouvant également être alkylé dans le cas de N ;
R² et R³ étant identiques ou différents, et représentant un radical alkyle en C₁-C₁₂ ou un radical aryle, les radicaux pouvant également être substitués ou pouvant être reliés l'un à l'autre par un pont alkylène ;
R⁴ représentant un groupe alkyle en C₁ à C₆ ou un groupe aryle non substitué ou substitué avec des radicaux alkyle en C₁ à C₃, les groupes pouvant également contenir des hétéroatomes ou des halogènes ; et
m correspondant à la valence du métal.

9. Procédé de fabrication d'un élément optique contenant les étapes suivantes :
a) la fabrication d'une composition contenant :
a1) au moins un polymère organique ; et
a2) au moins un complexe métallique de formule
X₍ₘ₋ₙ₎MR¹ₙ (I)
dans laquelle M représente Ti, Zr, Ta, V, Nb, Cr, Mo, W, Mn ou Re ;
R¹ est dérivé d'une α-hydroxycétone, d'un glyoxylate ou d'une aminocétone ;
X est un groupe sans groupe sensible à la lumière ;
n a une valeur d'au moins 1 à m et m correspond à la valence du métal ; et
a3) au moins un solvant ;
b) l'application de la composition sur une surface ou dans un moule ;
c) la génération d'une différence de potentiel pour la diffusion dirigée des complexes métalliques par décomposition locale des complexes métalliques.

10. Élément optique pouvant être obtenu à partir d'une composition selon l'une quelconque des revendications 1 à 8 ou d'un procédé selon la revendication 9.

11. Utilisation de l'élément optique selon la revendication 10 pour des applications holographiques, des lentilles planes à gradient d'indice dans l'optique d'imagerie, des films de gestion de la lumière, des diffuseurs, des systèmes d'affichage tête haute, des systèmes d'affichage tête basse, des fibres optiques et des mémoires de données optiques.
